# EUROPEAN PATENT SPECIFICATION

(11) **EP 2 864 524 B1**
(45) Date of publication and mention of the grant of the patent: **18.05.2016**
(21) Application number: 12762407.0
(22) Date of filing: 22.06.2012
(51) Int. Cl.: C25B 1/04, C25B 15/00, C25B 15/08, C07C 1/12, C10L 3/08

(54) **UNIT FOR THE ACCUMULATION OF ELECTRICAL ENERGY VIA PRODUCTION OF METHANE**
SAMMELEINHEIT FÜR ELEKTRISCHE ENERGIE ÜBER METHANPRODUKTION
UNITÉ D'ACCUMULATION D'ÉNERGIE ÉLECTRIQUE PAR LE BIAIS D'UNE PRODUCTION DE MÉTHANE

(43) Date of publication of application: 29.04.2015
(73) Proprietor: Capriccioli, Andrea, Rocca Priora (IT)
(72) Inventor: Capriccioli, Andrea, Rocca Priora (IT)
(74) Representative: Bosman, Cesare
(86) International application number: PCT/IT2012/000192
(87) International publication number: WO 2013/190581

(56) References cited:
- DE-U1-202011 005 536
- US-A- 5 060 629
- US-A- 5 505 824
- US-A- 5 711 770

## Description

### TECHNICAL FIELD

The present invention relates to a unit for the accumulation of electrical energy via production of methane.

In particular, the unit forming the subject of the present invention is able to use low-cost electrical energy deriving from any energy source, whether renewable or not, for producing, via high-efficiency hydrolysis and with a passive safety system, the hydrogen necessary for the synthesis of CH₄. The methane thus produced may be used for the deferred production of electrical energy on demand (i.e., when required by the grid).

The unit forming the subject of the present invention is in effect a non-conventional energy accumulator of variable capacity as a function of the CO₂ previously accumulated and the low-cost electrical energy absorbed at input. At output peak high-cost or on-demand electrical energy will be produced.

### BACKGROUND ART

With reference to scenarios foreseeable in the short-to-medium term, both the use of renewable sources for producing electrical energy to be introduced directly into the national grid and the production of energy via large (nuclear and thermoelectric) basic power plants will present considerable difficulties of regulation. In particular, when the power of the electrical energy coming from renewable sources exceeds the level of 15% of all the power installed, there may arise problems of instability of the grid.

To this problem there is to be added the fact that up to the present day it is not always possible to ensure an adequate availability of energy produced by renewable sources given a request on the part of the grid loads.

As regards major plants that generate basic electrical energy, low management capacity leads in given conditions to an excess availability of electrical energy (especially during nighttime hours). Said excess of energy necessarily entails both a low commercial value thereof (low cost) and the need for a non-productive disposal thereof. In fact, in certain cases it is even advisable to interrupt the production of energy (in particular, from sources such as wind sources, hydraulic or mini-hydraulic sources, solar sources at low latitudes) in so far as it is potentially dangerous for the stability of the grid itself.

The problems referred to above are leading to the development of appropriate smart grids, which attempt to balance demand and supply of energy in real time. Obviously, accumulation systems, which operate as filters, radically facilitate the task of future smart grids in so far as they offer the flexibility indispensable for a simpler, more effective, safer and more economic management, also at a local level, of the distribution grid.

In the past, the problem of energy storage has led to the development of accumulation systems that still enable provision of electrical energy on demand.

Said systems are principally articulated in three categories:
- The first, which is the one historically best studied and most widespread especially in Italy, is the system of accumulation of potential energy by pumping water from basins downstream to ones upstream.
- The second, which is still under development, is the accumulation of thermal energy using solar energy (i.e., by means of molten salts at a high temperature).
- The third is the accumulation of electrochemical energy using batteries of various types (from the old lead/sulphuric acid batteries to the more up-to-date ones, e.g., lithium-ion or sodium-sulphur NaS batteries). Other types (e.g. vanadium-oxide batteries) still seem to be in the embryonic state.

### DISCLOSURE OF INVENTION

The aim of the present invention is to provide a unit for the accumulation of electrical energy, the technical characteristics of which can be exploited in an efficient and intrinsically safe way low-cost electrical energy to convert it and accumulate it in the chemical energy of methane, which in turn may be used as such or for producing energy in a closed system when required by the grid.

The subject of the present invention is a unit for the accumulation of electrical energy, the essential characteristics of which are set forth in claim 1, and the preferred and/or auxiliary characteristics of which are set forth in claims 2-7.

### BRIEF DESCRIPTION OF THE DRAWINGS

For a better understanding of the invention described hereinafter are a number of embodiments provided purely by way illustrative and non-limiting example with the aid of the figures of the annexed plate of drawings, wherein:
Figure 1 illustrates schematically the device forming the subject of the present invention according to a first embodiment; and
Figure 2 illustrates schematically the device forming the subject of the present invention according to a second embodiment.

### BEST MODE FOR CARRYING OUT THE INVENTION

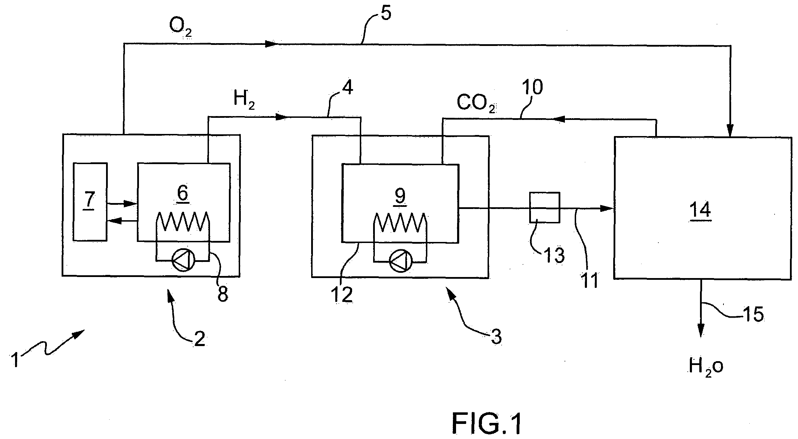

Designated as a whole by 1 in Figure 1 is the unit forming the subject of the present invention. The unit 1 comprises: an electrolyser 2 designed for carrying out electrolysis of water for the production of H₂; a methanation reactor 3 designed to produce CH₄ by means of the reaction between CO₂ and the H₂ produced by the electrolyser 2; a conduction line 4 for conveying the H₂ from the electrolyser 2 where it is produced to the methanation reactor 3, where it is used for the production of CH₄; and a line 5 for offtake of the O₂, which is also produced by the electrolyser 2.

The electrolyser 2 comprises: an electrolytic cell 6, housed in which are commercial electrodes or else porous electrodes charged with nanostructured catalyst so as to offer a vast electrolyte-electrode contact surface; and a tank 7 of water at an adjustable pressure, hydraulically connected to the electrolytic cell 6. As will be described hereinafter, the hydraulic connection between the electrolytic cell 6 and the tank 7 guarantees activity in conditions of safety of the unit forming the subject of the present invention.

The electrolyser 2 further comprises a system of thermal regulation of water 8, designed to regulate the temperature of water as electrolyte to be able to optimize the efficiency of the electrolytic process.

The methanation reactor 3 comprises a reaction chamber 9, which is appropriately thermally insulated and housed within which is a compact porous or granular solid support made of material with high thermal conductivity, which is charged with a methanation catalyst, such as, for example, Cu-Zn-Cr, Fe-Cu, Ni, Ru, etc.

The methanation reactor 3 is structured in such a way as to enable entry and diffusion of the two reaction gases (H₂ and CO₂) inside the catalyzed support and offtake of CH₄ as synthesis gas produced. In this regard, the unit 1 comprises a feed line 10 for supply of the CO₂ into the methanation reactor 3, and a line 11 for offtake of the CH₄ from the methanation reactor 3.

The two reaction gases are introduced into the methanation reactor 3 according to a minimum H₂/CO₂ ratio equal to 4, and diffuse inside the solid support, in so far as it is porous, for reacting in the presence of the catalyst and in the conditions of Pmin=2bar and Tmax=300°C. In this way, the CH₄ that is produced is drawn off by the offtake line 11.

As may appear obvious to a person skilled in the branch, on the line 11 for offtake of the CH₄ there operate separator means designed to separate the CH₄ produced from the unreacted H₂.

The methanation reaction that occurs in the methanation reactor 3 is the Sabatier reaction (I), which is not particularly burdensome in terms of operating conditions, given that it occurs at Pmin=200kPa and Tmax=300°C,

CO₂ + 4H₂ → CH₄ + 2H₂O (I)

ΔH_{298K}= -164.9 kJ/mol
ΔG_{298K}= -27 kcal/mol

In the aforesaid operating conditions, with a molar ratio of supply of H₂/CO₂ of 4, according to the relevant literature there is a reaction conversion of approximately 90% in traditional reactors.

If membranes selective for vapour integrated in the reactor were used, ideal yields of 100% could be reached. In particular, the methanation reactor 3 comprises membranes permeable only to hydrogen in such a way as to cause the non-reacted H₂ to recirculate in the reaction chamber 9 in order to optimize the yields.

The methanation reactor 3 further comprises a cooling system 12, which is necessary since, as emerges from the thermodynamic values referred to above, the reaction for producing CH₄ is exothermic. In this regard, the cooling system 12 is connected to a plurality of temperature sensors set inside the catalyzed support for regulating activity thereof effectively.

The line 11 for offtake of the CH₄ comprises a thermostatic safety valve 13, which, as a function of the temperature recorded, will obstruct or otherwise the passage of CH₄.

The thermostatic safety valve 13, together with the hydraulic connection of the electrolytic cell 6 with the tank 7 guarantees the intrinsic safety of the unit forming the subject of the present invention.

In fact, as a consequence of the partial or total closing by the thermostatic safety valve 13, there is an increase in the pressure of H₂ inside the electrolytic cell 6. Once the pressure inside the electrolytic cell 6 exceeds a pressure value previously set for the tank 7, there occurs a reflux of the electrolyte (water) from the electrolytic cell 6 to the tank 7. Said reflux causes a reduction of the level of the electrolyte in the electrolytic cell 6, with consequent reduction or interruption of the reaction of electrolysis, which means a reduction or interruption of the flow of H₂ as well as of the generation of heat.

Only a subsequent reduction of temperature and pressure inside the electrolytic cell 6 can bring back the electrolyte into the cell with consequent resumption of the production of H₂.

Preferably, the unit 1 can comprise means for producing electrical energy 14 by combustion of CH₄ with the use of the O₂ produced by the electrolyser 2 and drawn off by the offtake line 5. The CO₂ produced by combustion is re-used as reactive gas in the methanation reactor 3. The H₂O produced by combustion is drawn off by an offtake line 15 for being subsequently reintroduced into the tank 7 and being used in the electrolytic process. The use of the O₂ for deferred combustion of the methane produced and accumulated, the re-use of CO₂ and water, and a due accumulation of H₂ (e.g., maintenance of the methanizer 3) entail the presence of intermediate storage volumes that can be incorporated alternatively in the electrolyser 2 and in the methanation reactor 3 or in the means for producing electrical energy 14.

As may appear evident from the foregoing description, the unit forming the subject of the present invention enables accumulation of electrical energy by use thereof for the process of electrolysis and with subsequent synthesis of CH₄. The CH₄ produced may be either introduced into the methane grid for its traditional uses or used for producing once again, by combustion, electrical energy according to the grid demand. In this latter case, it should be pointed out that the CO₂ produced balances the CO₂ consumed in the methanation reaction, rendering the process as a whole CO₂-neutral.

The electrical energy necessary for hydrolysis can derive from any plant or from the distribution grid, whilst the CO₂ can come from combustion plants burning fossil sources or industrial processes that have CO₂ as waste by-product of their production activity.

It should moreover be emphasized how the unit forming the subject of the present invention is able to ensure the required levels of safety, guaranteeing automatic and simultaneous quenching of the methanation and hydrolysis reaction once the temperature of the methanation reactor has exceeded a pre-set threshold.

Finally, if the unit forming the subject of the present invention comprises the means for producing electrical energy 14 connected to the electrolyser 2 and to the methanation reactor 3 as described in terms of transfer of O₂ and CO₂ respectively, there is obtained a completely closed system in which only electrical energy enters and exits.

In a context of management of production and of supply of energy, the unit forming the subject of the present invention enables use of low-cost electrical energy or even of energy that is otherwise not producible, for converting it into the chemical energy of methane and so accumulating it and, when it becomes necessary because it is required by the grid, converting it again into electrical energy by means of a cycle that could be "closed" and certainly with CO₂-neutral.

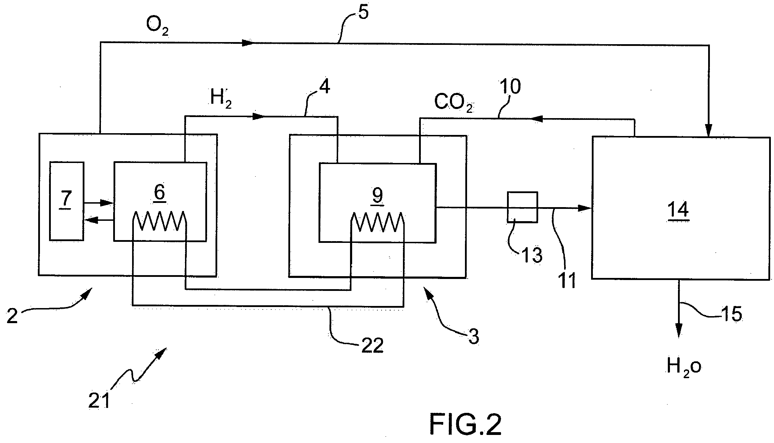

Designated by 21 in Figure 2 is a second embodiment of the unit forming the subject of the present invention. Parts that are the same as those of the unit 1 described above will be designated by the same numbers and will not be described again.

The unit 21 differs from the unit 1 basically in that it comprises a variable-thermal-conductivity device 22, which has the function of subtracting the heat produced from the methanation reactor 3 in a variable way according to the operating steps of the methanation reactor 3 itself. The heat subtracted from the methanation reactor 3 can be used for various purposes. In particular, according to what is illustrated in Figure 2, the heat subtracted from the methanation reactor 3 is transferred to the electrolytic cell 6 to optimize the electrolytic process in progress.

According to a preferred embodiment, the variable-thermal-conductivity device 22 comprises a heat exchanger constituted by a first plurality of metal plates thermally connected to said reaction chamber 9 and arranged in an alternating way with a second plurality of metal plates thermally connected with a fluid to which the heat is transferred. The thermal conductivity between the first plurality of metal plates and the second plurality of metal plates depends upon the extent in which these are immersed in a heat-conductive fluid. In other words, in the case where the heat-conductive fluid is not present between the metal surfaces, the reaction chamber 9 will be in a situation of almost thermal insulation, whereas the presence of heat-conductive fluid between the metal plates favours thermal conduction. Said thermal conduction depends upon the extent to which the plates are immersed in the conductive liquid and, hence, upon the amount of the conductive liquid itself.

The heat-conductive fluid has a flow rate that can be varied by the action of filling/emptying means in order to vary the thermal conductivity between the metal plates as a function of the temperature recorded inside the solid support in the reaction chamber. In this regard, the variable-thermal-conductivity device 22 is connected to a plurality of temperature sensors set inside the solid support. The second plurality of metal plates is thermally connected to a secondary fluid, which can transmit the heat received to the electrolyser 2 as well as to any other utilizer of heat.

In other words, the variable-thermal-conductivity device 22 constitutes an element of thermal coupling between the methanation reactor 3 and the electrolyser 2 and enables a coordinated management of the temperature in the electrolytic cell and in the methanation reactor, with the obvious advantages in terms of energy and economy that this entails.

## Claims

1. A unit (1; 21) for accumulation of energy, comprising an electrolyser (2), designed to carry out electrolysis of H₂O to produce H₂ and O₂, and a methanation reactor (3), designed to produce CH₄ by causing the H₂ produced by the electrolyser (2) to react with CO₂; said unit **being characterized in that** it comprises a direct conduction line for the H₂ (4) from the electrolyser (2) to the methanation reactor (3) and a thermostatic safety valve (13) arranged on a line (11) which is designed to draw off the CH₄ produced by the methanation reactor, and **in that** said electrolyser (2) comprises an electrolytic cell (6) and a tank (7) for supply of water hydraulically connected to the electrolytic cell (6) for supplying it with water as electrolyte; said electrolytic cell (6) and said tank (7) being connected to each other by a first conduit responsible for supplying water from the tank (7) to the electrolytic cell, and by a second conduit responsible for a reflux of water from the electrolytic cell (6) to the tank (7).

2. The unit (1; 21) for accumulation of energy according to Claim 1, **characterized in that** said methanation reactor (3) comprises a reaction chamber (9), which is appropriately thermally insulated and housed within which is a support made of material with high thermal conductivity, which is charged with a methanation catalyst; said support having a porous structure such as to enable H₂ and CO₂ to diffuse inside it so that they can react to form CH₄.

3. The unit (1; 21) for accumulation of energy according to Claim 2, **characterized in that** it comprises means for producing electrical energy (14) by combustion of CH₄ with the use of the O₂ produced by the electrolyser (2); said means for producing electrical energy (14) producing CO₂ to be introduced into the methanation reactor (3) and H₂O to be introduced into the tank (7) for being used in the electrolytic process.

4. The unit (21) for accumulation of energy according to any one of the preceding claims, **characterized in that** it comprises a variable-thermal-conductivity device (22), which has the function of subtracting heat from the methanation reactor (3).

5. The unit (21) for accumulation of energy according to Claim 4, **characterized in that** said variable-thermal-conductivity device (22) comprises a heat exchanger comprising a first plurality of metal plates thermally connected to said methanation reactor (3), a second plurality of metal plates arranged alternating with said first plurality of metal plates, and means for filling/emptying a heat-conductive fluid in such a way as to vary the level of immersion of said first and second pluralities of metal plates in said conductive fluid.

6. The unit (21) for accumulation of energy according to Claim 5, **characterized in that** said variable-thermal-conductivity device (22) comprises a secondary fluid set in thermal contact with said second plurality of metal plates and responsible for heating in the electrolytic cell (6).

7. The unit (21) for accumulation of energy according to Claim 6, **characterized in that** said variable-thermal-conductivity device (22) is connected to a plurality of temperature sensors set inside a solid support which is housed in said methanation reactor (3) and within the methanation reaction occurs.

## Patentansprüche

1. Einheit (1; 21) zur Akkumulation von Energie, mit einem Elektrolyseur (2), der so ausgebildet ist, dass dieser eine Elektrolyse von H₂O durchführt, um H₂ und O₂ zu erzeugen, und mit einem Methanisierungsreaktor (3), der so ausgebildet ist, dass dieser CH₄ erzeugt, indem dass durch den Elektrolyseur (2) erzeugte H₂ veranlasst wird, mit CO₂ zu reagieren; wobei die Einheit **dadurch gekennzeichnet ist, dass** diese aufweist eine direkte Verbindungsleitung für das H₂ (4) von dem Elektrolyseur (2) zu dem Methanisierungsreaktor (3) und ein auf einer Leitung (11) angeordnetes Thermostat Sicherheitsventil (13), welches dazu ausgelegt ist, dass durch den Methanisierungsreaktor erzeugte CH₄ abzuziehen, und dadurch, dass der Elektrolyseur (2) aufweist eine Elektrolysezelle (6) und einen Behälter (7) für eine Wasserversorgung, der mit der Elektrolysezelle (6) hydraulisch verbunden ist, um diese mit Wasser als Elektrolyt zu versorgen; wobei die Elektrolysezelle (6) und der Behälter (7) miteinander verbunden sind durch eine erste Leitung, die für ein Zuführen von Wasser aus dem Behälter (7) zu der Elektrolysezelle verantwortlich ist, und durch eine zweite Leitung, die für einen Rückfluss von Wasser aus der Elektrolysezelle (6) zum Behälter (7) verantwortlich ist.

2. Einheit (1; 21) zur Akkumulation von Energie nach Anspruch 1, **dadurch gekennzeichnet, dass** der Methanisieningsreaktor (3) eine Reaktionskammer (9) umfasst, welche in geeigneter Weise wärmeisoliert ist und innerhalb aufgenommen ist, welcher ein Träger ist, der aus einem Material mit hoher Wärmeleitfähigkeit hergestellt ist, welcher mit einem Methanisieningskatalysator beladen ist; wobei der Träger eine poröse Struktur aufweist, so dass dieser dem H₂ und O₂ ermöglicht, innerhalb diesem zu diffundieren, so dass sie reagieren können, um CH₄ zu bilden.

3. Einheit (1; 21) zur Akkumulation von Energie nach Anspruch 2, **dadurch gekennzeichnet, dass** diese eine Einrichtung zum Erzeugen elektrischer Energie (14) durch Verbrennung von CH₄ unter Verwendung des durch den Elektrolyseur (2) erzeugten O₂ umfasst; wobei die Einrichtung zum Erzeugen elektrischer Energie (14) erzeugt CO₂, das in den Methanisieningsreaktor (3) eingeleitet werden soll, und H₂O, das in dem Behälter (7) eingeleitet werden soll, um in dem Elektrolyseprozess verwendet zu werden.

4. Einheit (21) zur Akkumulation von Energie nach einem der vorhergehenden Ansprüche, **dadurch gekennzeichnet, dass** diese eine Einrichtung 22) variabler Wärmeleitfähigkeit umfasst, welche die Funktion hat, Wärme aus dem Methanisierungsreaktor (3) abzuziehen.

5. Einheit (21) zur Akkumulation von Energie nach Anspruch 4, **dadurch gekennzeichnet, dass** die Einrichtung (22) mit variabler Wärmeleitfähigkeit einen Wärmetauscher umfasst, der aufweist eine erste Mehrzahl von Metallplatten, die thermisch mit dem Methanisieningsreaktor (3) verbunden sind, eine zweite Mehrzahl von Metallplatten, die abwechselnd mit der ersten Mehrzahl von Metallplatten angeordnet sind, und eine Einrichtung zum Füllen/Leeren eines wärmeleitfähigen Fluids in der Weise, dass das Eintauchniveau der ersten und zweiten Mehrzahl von Metallplatten in das leitfähige Fluid variiert wird.

6. Einheit (21) zur Akkumulation von Energie nach Anspruch 5, **dadurch gekennzeichnet, dass** die Einrichtung (22) mit variabler Wärmeleitfähigkeit ein zweites Fluid umfasst, das in Wärmekontakt mit der zweiten Mehrzahl von Metallplatten gebracht ist und für das Erwärmen der Elektrolysezelle (6) verantwortlich ist.

7. Einheit (21) von Energie nach Anspruch 6, **dadurch gekennzeichnet, dass** die Einrichtung mit variabler Wärmeleitfähigkeit (22) mit einer Mehrzahl von Temperatursensoren verbunden ist, die innerhalb eines festen Trägers angebracht sind, welcher in dem Methanisierungsreaktor (3) aufgenommen ist und in welchem die Methanisierungsreaktion auftritt.

## Revendications

1. Unité (1 ; 21) d'accumulation d'énergie, comprenant un électrolyseur (2), conçu pour réaliser une électrolyse de H₂O pour produire du H₂ et de l'O₂, et un réacteur de méthanation (3), conçu pour produire du CH₄ en faisant réagir le H₂ produit par l'électrolyseur (2) avec du CO₂ ; ladite unité étant **caractérisée en ce qu'**elle comprend une ligne de conduction directe pour le H₂ (4) de l'électrolyseur (2) au réacteur de méthanation (3) et une soupape de sûreté thermostatique (13) agencée sur une ligne (11) qui est conçue pour évacuer le CH₄ produit par le réacteur de méthanation, et **en ce que** ledit électrolyseur (2) comprend une cellule électrolytique (6) et un réservoir (7) de fourniture d'eau hydrauliquement relié à la cellule électrolytique (6) pour lui fournir de l'eau en tant qu'électrolyte ; ladite cellule électrolytique (6) et ledit réservoir (7) étant reliés l'un à l'autre par une première conduite chargée de fournir l'eau du réservoir (7) à la cellule électrolytique, et par une seconde conduite en charge d'un reflux d'eau de la cellule électrolytique (6) au réservoir (7).

2. Unité (1 ; 21) d'accumulation d'énergie selon la revendication 1, **caractérisée en ce que** ledit réacteur de méthanation (3) comprend une chambre de réaction (9), qui est thermiquement isolée et reçue de manière appropriée à l'intérieur de laquelle se trouve un support fait d'un matériau ayant une conductivité thermique élevée, qui est chargé avec un catalyseur de méthanation ; ledit support ayant une structure poreuse de manière à permettre au H₂ et au CO₂ de se diffuser à l'intérieur de lui de sorte qu'ils puissent réagir pour former du CH₄.

3. Unité (1 ; 21) d'accumulation d'énergie selon la revendication 2, **caractérisée en ce qu'**elle comprend des moyens pour produire de l'énergie électrique (14) par combustion de CH₄ avec l'utilisation de l'O₂ produit par l'électrolyseur (2) ; lesdits moyens pour produire de l'énergie électrique (14) produisant du CO₂ devant être introduit dans le réacteur de méthanation (3) et du H₂O devant être introduit dans le réservoir (7) pour être utilisés dans le processus électrolytique.

4. Unité (21) d'accumulation d'énergie selon l'une quelconque des revendications précédentes, **caractérisée en ce qu'**elle comprend un dispositif à conductivité thermique variable (22), qui a pour fonction de soustraire de la chaleur du réacteur de méthanation (3).

5. Unité (21) d'accumulation d'énergie selon la revendication 4, **caractérisée en ce que** ledit dispositif à conductivité thermique variable (22) comprend un échangeur de chaleur comprenant une première pluralité de plaques métalliques thermiquement reliées audit réacteur de méthanation (3), une seconde pluralité de plaques métalliques agencées en alternance avec ladite première pluralité de plaques métalliques, et des moyens pour remplir/vider un fluide thermoconducteur de manière à faire varier le niveau d'immersion desdites première et seconde pluralités de plaques métalliques dans ledit fluide conducteur.

6. Unité (21) d'accumulation d'énergie selon la revendication 5, **caractérisée en ce que** ledit dispositif à conductivité thermique variable (22) comprend un fluide secondaire mis en contact thermique avec ladite seconde pluralité de plaques métalliques et en charge de chauffer dans la cellule électrolytique (6).

7. Unité (21) d'accumulation d'énergie selon la revendication 6, **caractérisée en ce que** ledit dispositif à conductivité thermique variable (22) est relié à une pluralité de capteurs de température placés à l'intérieur d'un support solide qui est reçu dans ledit réacteur de méthanation (3) et à l'intérieur duquel la réaction de méthanation se produit.
